# FASCICULE DE BREVET EUROPEEN

(11) **EP 0 665 951 B1**
(45) Date de publication et mention de la délivrance du brevet: **22.01.1997**
(21) Numéro de dépôt: 93922985.2
(22) Date de dépôt: 13.10.1993
(51) Int. Cl.: G01N 21/89

(54) **PROCEDE ET DISPOSITIF D'INSPECTION DE MATERIAU TRANSPARENT**
VORRICHTUNG UND VERFAHREN ZUR INSPEKTION TRANSPARENTEN MATERIALS
PROCESS AND DEVICE FOR INSPECTION OF A TRANSPARENT MATERIAL

(30) Priorité: 20.10.1992 FR 9212526
(43) Date de publication de la demande: 09.08.1995
(73) Titulaire: THOMSON multimedia, 92400 Courbevoie (FR)
(72) Inventeur: JUTARD, Yann, F-92402 Courbevoie Cédex (FR); SACRE, Jean-Jacques, F-92402 Courbevoie Cédex (FR)
(74) Mandataire: Ruellan-Lemonnier, Brigitte
(86) Numéro de dépôt international: FR9301015
(87) Numéro de publication internationale: WO9409358

(56) Documents cités:
- EP-A- 0 315 697
- DE-A- 3 926 349
- US-A- 3 814 946
- PATENT ABSTRACTS OF JAPAN vol. 011, no. 037 (P-543)4 Février 1987 & JP,A,61 207 953 (NEC CORP) 16 Septembre 1986
- PATENT ABSTRACTS OF JAPAN vol. 011, no. 211 (P-594)9 Juillet 1987 & JP,A,62 032 345 (YASKAWA ELECTRIC MFG CO) 12 Février 1987

## Description

La présente invention concerne un procédé et un dispositif d'inspection de matériau transparent et se rapporte au domaine de l'inspection industrielle automatique par imagerie en sortie de chaîne de fabrication.

D'une manière générale, l'inspection d'un matériau transparent tel que le verre, par exemple, a pour objet la détection et la localisation de défauts, appelés également "inclusions", générés lors de la phase de fabrication du verre. La répercussion de ces défauts sur la qualité du verre produit dépend de leur nombre, de leur forme et des normes imposées au verrier selon l'application visée.

Ces défauts sont de plusieurs types, dont les principaux sont du type bouillon et du type pierre :

Dans le type bouillon, les défauts sont des bulles gazeuses restées prisonnières dans le matériau en fusion et dont la forme varie de la sphère à un filament très allongé avec un rapport d'élongation supérieur à dix.

Dans le type pierre, les défauts sont des défauts d'homogénéité des constituants de base, entraînant la formation d'une particule visible dans le verre.

La taille et la forme de ces inclusions varient suivant le matériau et la profondeur dans le matériau. Leur taille est en générale inférieure au millimètre, la résolution maximale atteignant le dixième de millimètre.

L'analyse automatique de la qualité du matériau soit la détection automatique des défauts du matériau réagit directement sur sa production. Cette analyse permet de rejeter les produits défectueux mais aussi de disposer de statistiques fiables sur le nombre et la taille des défauts afin de modifier en conséquence les différents paramètres de production du matériau.

Des dispositifs connus d'inspection utilisent un système d'observation comportant une source lumineuse par exemple une source laser, illuminant un matériau transparent à inspecter. Le rayonnement lumineux, après passage dans le matériau est analysé par un dispositif de détection sensible aux rayonnements lumineux, par exemple une caméra CCD, et permet la détection et la localisation en profondeur de défauts éventuels.

Cependant l'utilisation de la vision artificielle pour l'inspection des matériaux transparents et du verre en particulier, est limitée actuellement par un manque d'efficacité concernant la discrimination entre des perturbations de surface, telles que des poussières déposées sur la surface du matériau à inspecter, et des inclusions qui sont des défauts réels de fabrication noyés dans la masse du matériau. En effet, la présence de poussières sur la surface du matériau est à l'origine de confusion quand un défaut réel est localisé dans la masse du matériau à proximité de la surface. Les poussières s'illuminent à proximité de la source lumineuse, et peuvent masquer un défaut éventuel situé à proximité de la surface.

D'autre part, certains défauts de forme allongée ne brillent pas lors de leur rencontre avec le faisceau lumineux et ne sont donc pas détectés.

Des dispositifs tels que décrits dans le volume 011, n° 037(P-543) du 4 février 1987 du document "PATENT ABSTRACTS OF JAPAN", utilisent un balayage rasant d'un faisceau laser et contrôlent la dispersion du faisceau laser par un dépôt en surface du verre pour le discriminer d'une inclusion. Ces dispositifs de discrimination sont de réalisation complexe car nécessitent l'utilisation spécifique d'un laser et des détecteurs adaptés pour la prise en compte de la déviation du faisceau.

Le but de l'invention est de pallier les inconvénients précités.

A cet effet l'invention à pour objet un procédé d'inspection de matériau transparent par illumination au moyen d'au moins une source lumineuse, et par observation de l'épaisseur du matériau par au moins une caméra, caractérisé en ce qu'il consiste à éclairer uniformément un fond clair placé relativement à la caméra derrière le matériau pour être visualisé par transparence au travers du matériau, couvrant le champ visuel de la caméra et servant de référence de contraste, à éclairer latéralement la surface du matériau pour distinguer des défauts inclus dans le matériau d'éléments parasites déposés sur sa surface, à visualiser par transparence par la caméra, placée à la verticale de la surface du matériau, une séquence d'images contrastées reproduisant l'épaisseur du matériau, et à traiter des informations acquises par les images successives représentatives du matériau vu dans son épaisseur pour détecter et localiser les défauts inclus dans l'épaisseur du matériau.

L'invention a également pour objet un dispositif d'inspection destiné à la mise en oeuvre du procédé tel que décrit ci-dessus.

L'invention a également pour avantage de discriminer les défauts noyés dans la masse du matériau à proximité de la surface des éléments parasites de surface, tels que des poussières.

L'invention a également pour avantage d'utiliser des moyens communs au contrôle du matériau transparent et à la discrimination des défauts noyés dans la masse du matériau des éléments parasites de surface, et ainsi de faire appel à un dispositif simple. Elle a aussi pour avantage de localiser les défauts quelles que soit leur forme et leur orientation et de mesurer leur profondeur dans le matériau par l'exploitation d'un faisceau laser sans nécessiter de détecteurs supplémentaires.

D'autres caractéristiques et avantages de l'invention apparaîtront ci-après à l'aide de la description qui suit faite en regard des dessins annexés qui représentent
- la figure 1, une vue générale d'un dispositif d'inspection selon l'invention ;
- la figure 2, une vue latérale d'un mode de réalisation du dispositif selon l'invention ;
- la figure 3, une vue de détail du dispositif selon l'invention, et
- la figure 4, un schéma optique.

Sur ces figures, les éléments homologues sont désignés par le même repère.

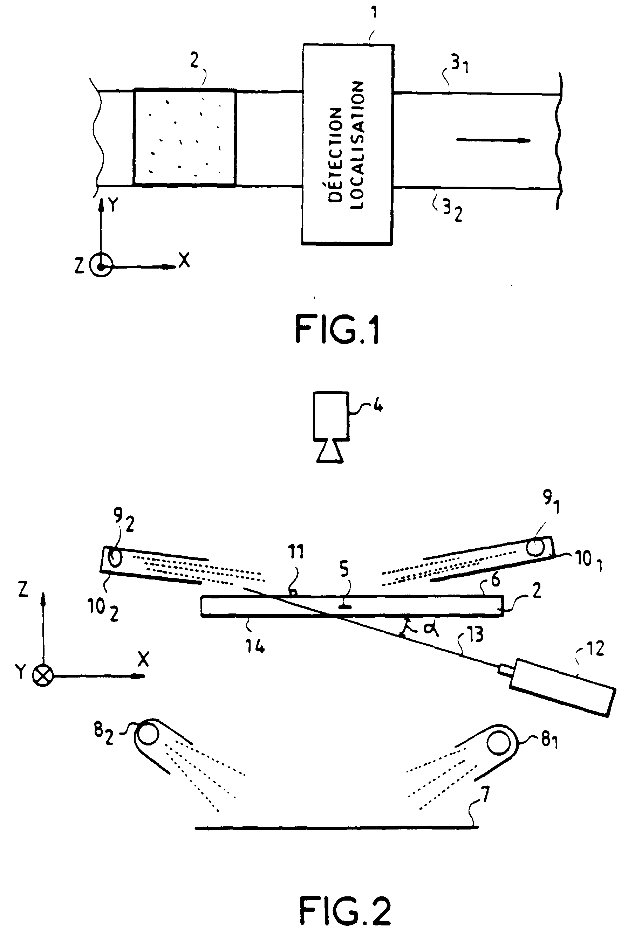

Le dispostif d'inspection selon l'invention comporte, dans un mode de réalisation illustré schématiquement en vue de dessus sur la figure 1, un poste 1 de détection et de localisation des défauts dans les trois dimensions, X, Y, Z. Un matériau transparent 2 est posé sur un convoyeur, constitué par exemple de deux rails parallèles 3₁ et 3₂ supportant le matériau 2, et il est inspecté au fur et à mesure qu'il passe à la verticale du poste 1 de détection et de localisation de défauts, le déplacement du convoyeur s'effectuant suivant l'axe X dans le sens indiqué par la flèche. Le poste 1 fournit une ou plusieurs images non représentées des coupes successives du matériau 2.

Une vue latérale d'un mode de réalisation du dispositif selon l'invention est illustrée par la figure 2. Sur cette figure le convoyeur 3₁ et 3₂ n'est pas représenté. Le dispositif comporte une caméra 4 noir et blanc utilisant par exemple des capteurs de type CCD, observant par transparence le matériau 2 à inspecter. Suivant l'optique qui lui est adjointe, la caméra 4 couvre une zone variable du matériau avec une résolution donnée. Le réglage de l'objectif de la caméra 4 fait l'objet d'un compromis entre la profondeur de champ couvrant l'épaisseur du matériau 2 de l'ordre de quelques millimètres et le grandissement assurant la détection des défauts 5 avec une résolution maximale de l'ordre du dixième de millimètre.

La caméra 4 est placée à la perpendiculaire de la surface supérieure 6 du matériau 2.

Un fond clair 7 faiblement éclairé est placé en retrait sous le matériau 2. La caméra 4 couvre la zone d'observation du matériau correspondant à une coupe dans l'épaisseur du matériau 2 et ne reçoit pas de lumière directe issue de l'éclairage du fond clair 7. Pour obtenir un éclairage uniforme sur le fond 7, des tubes néons, 8₁ et 8₂ par exemple, sont placés autour du fond clair 7 qui diffuse ainsi la lumière reçue sur sa surface. Il peut être réalisé par exemple en papier blanc. Ce fond clair 7 réalise en fait un plan dont le contraste uniforme sert de référence de contraste. Ainsi, dans l'exemple dit fond en papier blanc, la caméra 4 reproduit une image entièrement blanche correspondant à une coupe du matériau transparent 2 sans défaut. Par contre tout défaut 6 présent dans la masse du matériau 2 est perçu comme une tâche sombre sur le fond clair 7.

Une source d'éclairage à faisceau rasant 9₁ et 9₂, par exemple de lumière blanche, est disposée latéralement au matériau 2. L'orientation des rayons lumineux incidents sur la surface supérieure 6 du matériau 2 est déterminée de telle sorte qu'ils ne pénètrent quasiment pas dans le matériau 2 évitant ainsi d'illuminer des défauts 5 inclus à proximité de la surface 6. L'éclairage est par exemple réalisé par des tubes néons de faible puissance. Ils sont placés dans un dispositif 10₁ et 10₂ tel que les rayons lumineux sortent tous pratiquement parallèles les uns avec les autres. Le dispositif 10₁ et 10₂ est par exemple constitué de deux surfaces planes mises en parallèle servant de guide optique pour les rayons lumineux. Cet éclairage a pour but d'éclairer la surface supérieure 6 du matériau 2. Ainsi tout ce qui se trouve en surface dans le champ visuel de la caméra 4 est éclairé et renvoie une surintensité lumineuse vers la caméra 4.

L'éclairage rasant peut également utiliser une source lumineuse dont la longueur d'onde des rayons incidents délivrés par la source est telle qu'ils ne pénètrent pas dans le matériau d'indice de réfraction donné. Auquel cas, les détecteurs de la caméra sont adaptés pour détecter ces longueurs d'ondes. Des poussières 11 déposées sur la surface supérieure 6 du matériau 2 se traduisent sur l'image reconstituée par la caméra 4, soit comme des points plus clairs par exemple, blancs, se détachant du fond clair 7, pouvant être légèrement grisâtre servant de référence de contraste, soit noyés dans le fond 7.

Une source lumineuse 12 de type laser, par exemple à l'hélium néon est placée sous le matériau 2 à inspecter centré entre les deux rails 3₁ et 3₂ entre le matériau 2 et le fond clair 7, et émet un faisceau 13 formant un angle déterminé, α, entre la face inférieure 14 du matériau 2 et le faisceau 13. La source laser 12 est couplée à un moyen connu d'expansion de faisceau non représenté. Ce moyen est par exemple constitué d'un barreau optique de façon à épandre le faisceau laser 13 émis par la source 12 dans une seule direction. La disposition inclinée de la source laser 12 par rapport à la face inférieure 14 du matériau 2 permet de n'utiliser qu'une seule caméra 4 dans le cas ou le matériau 2 n'est pas plan mais légèrement convexe.

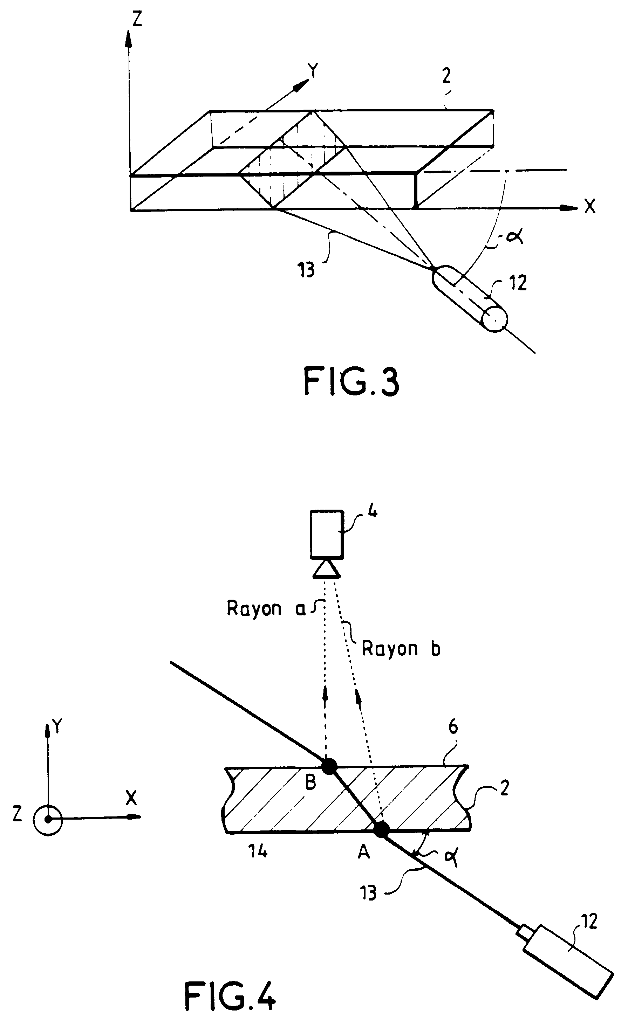

La figure 3 représente une vue partielle en perspective du mode de réalisation précédent représentant, dans le référentiel X-Y-Z, le matériau 2 éclairé par la source laser 12. Le faisceau laser 13 ainsi conformé par les moyens optiques précédents forme un rideau lumineux, représenté par une zone hachurée, d'épaisseur adaptée, qui correspond à chaque instant à une coupe oblique du matériau 2.

La figure 4 illustre une coupe partielle du matériau 2 et la position du laser 12 par rapport au matériau 2. Le faisceau laser 13 émis par la source laser 12 traverse de part en part l'épaisseur du matériau transparent 2, et forme un angle α avec la face inférieure 14 du matériau 2. Chaque face 6 et 14 du matériau 2 ainsi traversée par le faisceau laser 13 respectivement aux points A et B est traduite sur l'image par un trait horizontal, ou une ligne courbe suivant la courbure du matériau, en surbrillance par rapport au fond 7. La distance entre chacun de ces traits sert de référence pour le calcul de la profondeur des défauts 5 dans l'épaisseur du matériau 2 délimité par les deux traits. Ainsi la hauteur du plan de coupe du matériau visualisé par la caméra 4 dans le matériau 2 est donnée par le segment AB représenté comme l'intersection du faisceau laser 13 avec le matériau transparent 2. Les rayons a et b sont représentés sur la figure 4 par des lignes en pointillés et sont les rayons, issus du faisceau laser 13, générés par diffusion à chaque interface air/matériau, 13, et matériau/air, 6, et détecté par la caméra 4. Ainsi diffusés ils évitent un éblouissement de la caméra 4. D'autre part, cette disposition sans toutefois trop absorber le rayonnement, permet d'éviter des réflexions multiples dans le spectre visible. L'inclinaison de l'angle α de la source laser 12 permet en outre d'augmenter la résolution de la mesure en profondeur des défauts 5, la segment AB étant plus long comparé à un segment coupant verticalement l'épaisseur du matériau 2. L'angle α est, par exemple, de 45°, au delà, la résolution sur l'erreur relative de la mesure de la profondeur du défaut 5 diminue.

Les informations fournies par le poste 1 permettent une localisation par imagerie des défauts dans les trois dimensions X, Y et Z. L'information de luminance dit défaut 5 permet en outre de quantifier approximativement sa taille.

Avec une caméra 4 fonctionnant une cadence de 25 images par seconde et une vitesse maximale de défilement uniforme de 5 cm par seconde, le défaut 5 se déplace donc de 2 mm par image.

La vitesse de déplacement uniforme est liée à la fréquence d'acquisition des images par la caméra 4 et un compromis avec les différents éclairages mis en jeu dans la présente invention permet d'optimiser la qualité des images.

Les images acquises lors du déplacement du matériau transparent 2 porté par le convoyeur 3₁ et 3₂ par rapport au poste de détection et de localisation 1 permet, d'une image à l'autre, d'extraire les informations utiles à l'analyse :

la luminance permet de séparer les défauts 5 représentés par des tâches sombres du fond clair 7 servant de référence, les coordonnés X, Y des pixels représentatifs d'un défaut 5 donc d'une tâche sombre, permettent de déterminer la position d'un défaut 5 dans l'image, le déplacement de ces pixels sur une séquence de plusieurs images, par exemple une dizaine, permettent une corrélation des informations présentes sur ces images, et les surintensités se produisant lors de la rencontre du faisceau laser 13 avec un défaut 5 permettent le calcul par un calculateur non représenté de sa profondeur dans le matériau 2.

Pour séparer les tâches sombres correspondant aux défauts 5 du fond clair 7 ainsi qu'éventuellement les surbrillances dues aux poussières 11, le calculateur utilise par exemple une méthode de calcul par seuil adaptatif et/ou de détection de contour.

D'autre part, à partir des multiples informations recueillies, le procédé effectue un suivi d'un défaut 5 dans l'épaisseur du matériau 2 représenté par un point sombre puis brillant lors du passage du défaut 5 dans le faisceau laser 13, et des poussières 11, représentées par des points plus clairs que le fond 7 ou noyés dans le contraste uniforme du fond 7, d'une image à l'autre. Ainsi l'exploitation de la redondance d'informations rend plus fiable la détection des défauts 5.

Le traitement d'image effectuée par le calculateur est alors localement focalisé sur la zone proche présumée entourant le défaut 5, pour prendre en compte sa forme, sa luminance etc... L'analyse fine de cette zone utilise, par exemple, un algorithme de prédiction utilisant une méthode de corrélation statistique.

L'invention n'est pas limitée au procédé et au dispositif d'inspection tel que décrit en détail ci-dessus. En particulier, il a été décrit un convoyeur sur lequel est entraîné un matériau transparent à inspecter qui défile à la verticale d'un poste d'observation. Mais, il est possible d'utiliser, sans sortir du cadre de l'invention, un poste d'observation mobile, le matériau à inspecter restant fixe. Cette disposition est notamment intéressante pour les grosses pièces à inspecter.

De plus, l'éclairage rasant de lumière blanche, et l'éclairage du fond clair peuvent être réalisés à partir de sources lumineuses de nature différente de celles de l'invention, de même pour le type de capteur utilisé par la caméra ; une contrainte importante à respecter étant de fournir un éclairage global permettant à la caméra de distinguer un élément parasite de surface d'une inclusion.

Enfin, il est tout à fait possible, sans sortir du cadre de l'invention de coupler au poste de détection et de localisation un deuxième poste identique tête-bêche de façon à détecter des éléments parasites de surface déposés sur la face inférieure du matériau.

## Revendications

1. Procédé d'inspection de matériau transparent (2) par illumination au moyen d'au moins une source lumineuse, et par observation de l'épaisseur du matériau (2) par au moins une caméra (4), caractérisé en ce qu'il consiste à éclairer uniformément un fond clair (7) placé relativement à la caméra (4) derrière le matériau (2) pour être visualisé par transparence au travers du matériau (2), couvrant le champ visuel de la caméra (4) et servant de référence de contraste, à éclairer latéralement la surface du matériau (2) pour distinguer des défauts (5) inclus dans le matériau (2) d'éléments parasites (11) déposés sur sa surface et diffractant la lumière latérale, à visualiser par transparence par la caméra (4), placée à la verticale de la surface du matériau (2), une séquence d'images contrastées reproduisant l'épaisseur du matériau (2), et à traiter des informations acquises par les images successives représentatives du matériau (2) vu dans son épaisseur pour détecter et localiser les défauts (5) inclus dans l'épaisseur du matériau (2) correspondant à des tâches sombres sur l'image.

2. Procédé selon la revendication 1, caractérisé en ce qu'il consiste en outre, à illuminer le matériau par un faisceau laser (13) traversant le matériau (2) dans son épaisseur, incliné par rapport à sa surface et balayé pour former un rideau lumineux plan et oblique, dans le champ visuel de la caméra (4) et à visualiser par transparence par la caméra (4) une séquence d'images contrastées reproduisant des coupes successives du matériau (2).

3. Procédé selon la revendication 2, caractérisé en ce qu'il consiste pour traiter les informations acquises par les images restituées par la caméra (4), à corréler les tâches sombres correspondant aux défauts (5) inclus dans l'épaisseur du matériau (2) avec les surintensités lumineuses produites par le faisceau laser rencontrant les inclusions.

4. Procédé selon la revendication 2 ou 3, caractérisé en ce qu'il consiste à calculer la profondeur des défauts (5) illuminés par le faisceau laser (13) à partir de leur emplacement relativement aux 2 traits lumineux formés par l'intersection du faisceau laser avec le matériau transparent (2) pour localiser les défauts dans les 3 dimensions.

5. Procédé selon l'une quelconque des revendications 1 à 4, caractérisé en ce qu'il consiste pour visualiser les coupes successives du matériau (2), à déplacer le matériau (2) sur un convoyeur (3₁, 3₂) suppostant le matériau (2) dans un plan horizontal (XY) sous un poste fixe de détection et de localisation (1).

6. Procédé selon la revendication 5, caractérisé en ce qu'il consiste en outre, à coupler au premier poste fixe (1) un deuxième poste identique tête-bêche pour visualiser les éléments parasites déposés de part et d'autre du matériau transparent (2).

7. Procédé selon l'une quelconque des revendications précédentes, caractérisé en ce qu'il consiste à corréler les informations de position des défauts acquises sur plusieurs images.

8. Dispositif pour la mise en oeuvre du procédé selon l'une quelconque des revendications 1 à 7, caractérisé en ce qu'il comporte au moins un poste de détection et de localisation (1) comportant une caméra (4) placée à la verticale du matériau (2) à inspecter pour visualiser une séquence d'images représentant les coupes successives dans l'épaisseur du matériau (2), un fond clair (7) uniformément éclairé, placé relativement à la caméra (4) derrière le matériau (2) couvrant le champ visuel de la caméra (4), une source d'éclairage latéral (9₁, 9₂) au matériau (2) dont le faisceau lumineux est réfléchi par la surface du matériau (2), une source d'éclairage de type laser (12) couplée à des moyens d'expansion du faisceau dans une seule direction pour former un rideau lumineux oblique destiné à éclairer des plans de coupe successifs dans l'épaisseur du matériau (2), et un calculateur de traitement d'images pour détecter et localiser les défauts (5) dans l'épaisseur du matériau (2).

9. Dispositif selon la revendication 8, caractérisé en ce que la source d'éclairage de type laser (12) est placée relativement à la caméra (4) derrière le matériau (2) pour former le rideau lumineux oblique.

10. Dispositif selon l'une quelconque des revendications 8 et 9, caractérisé en ce que le calculateur comporte un dispositif de calcul par seuil adaptatif et/ou de détection de contour pour séparer les tâches sombres du fond clair (7).

11. Dispositif selon l'une quelconque des revendications 8 à 10, caractérisé en ce que le calculateur comporte un dispositif de prédiction pour la détection de défauts (5) sur les images suivantes en utilisant une méthode de corrélation statistique.

## Patentansprüche

1. Verfahren zur Inspektion transparenten Materials (2) durch Beleuchtung mittels mindestens einer Lichtquelle und durch Betrachtung der Dicke des Materials (2) durch mindestens eine Kamera (4), dadurch gekennzeichnet, daß es darin besteht, einen hellen Hintergrund (7), der von der Kamera (4) aus gesehen hinter dem Material (2) angeordnet ist, um mittels Transparenz durch das Material (2) hindurch sichtbar gemacht zu werden, der das Blickfeld der Kamera (4) ausfüllt und als Kontrast-Bezugselement dient, gleichmäßig zu beleuchten, die Oberfläche des Materials (2) seitlich zu beleuchten, um im Material (2) eingeschlossene Fehler (5) von auf seiner Oberfläche abgelagerten und das seitliche Licht beugenden störenden Elementen (11) zu unterscheiden, eine Folge von kontrastreichen Bildern, welche die Dicke des Materials (2) wiedergeben, durch Transparenz durch die senkrecht zur Oberfläche des Materials (2) angeordnete Kamera (4) sichtbar zu machen, und Informationen, die durch die für das in seiner Dicke betrachtete Material (2) repräsentativen aufeinanderfolgenden Bilder erhalten wurden, zu verarbeiten, um die in der Dicke des Materials (2) eingeschlossenen Fehler (5), welche dunklen Flecken auf dem Bild entsprechen, zu erkennen und zu lokalisieren.

2. Verfahren nach Anspruch 1, dadurch gekennzeichnet, daß es außerdem darin besteht, das Material durch einen das Material (2) in seiner Dicke durchquerenden Laserstrahl (13) zu beleuchten, der relativ zu seiner Oberfläche geneigt ist und abgelenkt wird, so daß er im Blickfeld der Kamera (4) einen ebenen und schrägen Lichtvorhang bildet, und durch die Kamera (4) mittels Transparenz eine Folge von kontrastreichen Bildern sichtbar zu machen, welche aufeinanderfolgende Schnitte des Materials (2) wiedergeben.

3. Verfahren nach Anspruch 2, dadurch gekennzeichnet, daß es darin besteht, daß, um die Informationen zu verarbeiten, die durch die von der Kamera (4) wiedergegebenen Bilder gewonnen werden, eine Beziehung zwischen den dunklen Flecken, welche den in der Dicke des Materials (2) eingeschlossenen Fehlern (5) entsprechen, und den erhöhten Lichtstärken, die von dem auf die Einschlüsse auftreffenden Laserstrahl erzeugt werden, hergestellt wird.

4. Verfahren nach Anspruch 2 oder 3, dadurch gekennzeichnet, daß es darin besteht, die Tiefe der von dem Laserstrahl (13) beleuchteten Fehler (5) ausgehend von ihrer Lage relativ zu den 2 Lichtstrichen, die durch den Schnitt des Laserstrahl mit dem transparenten Material (2) gebildet werden, zu berechnen, um die Fehler in den 3 Dimensionen zu lokalisieren.

5. Verfahren nach einem der Ansprüche 1 bis 4, dadurch gekennzeichnet, daß es darin besteht, daß, um die aufeinanderfolgenden Schnitte des Materials (2) sichtbar zu machen, das Material (2) auf einer das Material (2) tragenden Fördereinrichtung (3₁, 3₂) in einer waagerechten Ebene (XY) unter einer feststehenden Station zur Erkennung und Lokalisierung (1) bewegt wird.

6. Verfahren nach Anspruch 5, dadurch gekennzeichnet, daß es außerdem darin besteht, an die erste feststehende Station (1) eine zweite, identische Station anzukoppeln, wobei sich das Oberteil der einen Station jeweils neben dem Unterteil der anderen befindet, um die störenden Elemente sichtbar zu machen, die sich auf beiden Seiten des transparenten Materials (2) abgelagert haben.

7. Verfahren nach einem der vorhergehenden Ansprüche, dadurch gekennzeichnet, daß es darin besteht, die aus mehreren Bildern gewonnenen Informationen über die Position der Fehler zueinander in Beziehung zu setzen.

8. Vorrichtung für die Durchführung des Verfahrens nach einem der Ansprüche 1 bis 7, dadurch gekennzeichnet, daß sie wenigstens eine Station zur Erkennung und Lokalisierung (1) umfaßt, welche folgendes umfaßt: eine Kamera (4), die senkrecht zu dem zu inspizierenden Material (2) angeordnet ist, um eine Folge von Bildern sichtbar zu machen, welche die aufeinanderfolgenden Schnitte in der Dicke des Materials (2) darstellen, einen gleichmäßig beleuchteten hellen Hintergrund (7), der von der Kamera (4) aus gesehen hinter dem Material (2) angeordnet ist und das Blickfeld der Kamera (4) ausfüllt, eine Quelle für die seitliche Beleuchtung (9₁, 9₂) des Materials (2), deren Lichtstrahl von der Oberfläche des Materials (2) reflektiert wird, eine Beleuchtungsquelle vom Laser-Typ (12), die an Mittel zur Aufweitung des Strahls in einer einzigen Richtung au gekoppelt ist, so daß ein schräger Lichtvorhang gebildet wird, der dazu bestimmt ist, aufeinanderfolgende Schnittebenen in der Dicke des Materials (2) zu beleuchten, und einen Rechner zur Verarbeitung von Bildern, um die Fehler (5) in der Dicke des Materials (2) zu erkennen und zu lokalisieren.

9. Vorrichtung nach Anspruch 8, dadurch gekennzeichnet, daß die Beleuchtungsquelle vom Laser-Typ (12) von der Kamera (4) aus gesehen hinter dem Material (2) angeordnet ist, um den schrägen Lichtvorhang zu bilden.

10. Vorrichtung nach einem der Ansprüche 8 oder 9, dadurch gekennzeichnet, daß der Rechner eine Vorrichtung zur Berechnung mittels adaptiver Schwelle und/oder Konturerkennung umfaßt, um die dunklen Flecken von dem hellen Hintergrund (7) zu trennen.

11. Vorrichtung nach einem der Ansprüche 8 bis 10, dadurch gekennzeichnet, daß der Rechner eine Vorhersagevorrichtung für die Erkennung von Fehlern (5) auf den nachfolgenden Bildern unter Anwendung eines Verfahren der statistischen Korrelation umfaßt.

## Claims

1. Method for inspecting transparent material (2) by illumination by means of at least one light source, and by observation of the thickness of the material (2) by at least one camera (4), characterized in that it consists in uniformly illuminating a bright background (2) placed relative to the camera (4) behind the material (2) to be viewed by transparency through the material (2), covering the field of view of the camera (4) and serving as contrast reference, in laterally illuminating the surface of the material (2) in order to distinguish defects included within the material (2) from parasitic elements (11) deposited on its surface and diffracting the lateral light, in viewing by transparency by the camera (4), placed in the vertical to the surface of the material (2), a sequence of contrasted images reproducing the thickness of the material (2), and in processing information acquired by the successive images which are representative of the material (2) seen in its thickness, to detect and locate the defects (5) included within the thickness of the material (2) corresponding to dark spots on the image.

2. Method according to Claim 1, characterized in that it further consists in illuminating by a laser beam (13) passing through the material (2) in its thickness and inclined in relation to its surface and scanned to form a plane and oblique light curtain, in the field of view of the camera (4) and in viewing by transparency, by the camera (4) , a sequence of contrasted images reproducing successive sections of the material (2).

3. Method according to Claim 2, characterized in that, in order to process the information acquired by the images reconstructed by the camera (4), it consists in correlating the dark spots corresponding to the defects (5) included within the thickness of the material (2) with the excess light intensities produced by the laser beam encountering the inclusions.

4. Method according to Claim 2 or 3, characterized in that it consists in calculating the depth of the defects (5) illuminated by the laser beam (13) on the basis of their location relative to the 2 light strokes formed by the intersection of the laser beam with the transparent material (2) to locate the defects in the 3 dimensions.

5. Method according to any one of Claims 1 to 4, characterized in that, in order to view the successive sections of the material (2), it consists in moving the material (2) on a conveyor (3₁, 3₂) supporting the material (2) in a horizontal plane (XY) beneath a fixed detection and location station (1).

6. Method according to Claim 5, characterized in that it further consists in coupling an identical second station to the fixed first station (1) in head to tail fashion in order to view the parasitic elements deposited on both sides of the transparent material (2).

7. Method according to any one of the preceding claims, characterized in that it consists in correlating the information on position of the defects which is acquired over several images.

8. Device for implementing the method according to any one of Claims 1 to 7, characterized in that it includes at least one detection and location station (1) including a camera (4) placed in the vertical to the material (2) to be inspected, to view a sequence of images representing the successive sections within the thickness of the material (2), a uniformly illuminated bright background (7) placed relative to the camera (4) behind the material (2) covering the field of view of the camera (4), an illumination source (9₁, 9₂) lateral to the material (2), the light beam of which is reflected by the surface of the material (2) towards the camera (4), a laser-type illumination source (12) coupled means for expanding the beam in one single direction to form an oblique light curtain intended to illuminate successive sectional planes within the thickness of the material (2), and an image-processing computer for detecting and locating the defect (5) within the thickness of the material (2).

9. Device according to Claim 8, characterized in that the laser-type illumination source (12) is placed relative to the camera (4) behind the material (2) in order to form the oblique light curtain.

10. Device according to either one of Claims 8 and 9, characterized in that the computer includes a device for calculating by adaptive threshold and/or for contour detection to separate the dark spots from the bright background (7).

11. Device according to any one of Claims 8 to 10, characterized in that the computer includes a prediction device for the detection of defects (5) on the following images by using a statistical correlation method.
